# EUROPEAN PATENT APPLICATION

(11) **EP 4 338 840 A1**
(43) Date of publication of application: **20.03.2024**
(21) Application number: 22806830.0
(22) Date of filing: 12.05.2022
(51) Int. Cl.: B01L 3/00

(54) **SAMPLE PROCESSING APPARATUS AND SAMPLE PROCESSING METHOD**

(30) Priority: 12.05.2021 WO PCT/CN2021/093357
(71) Applicant: Coyote Bioscience Co., Ltd., Beijing 100085 (CN)
(72) Inventor: LI, Xiang, Beijing 100101 (CN); HAN, Yuguang, Chengde, Hebei 068350 (CN); FENG, Huiying, Beijing 100088 (CN)
(74) Representative: J A Kemp LLP
(86) International application number: PCT/CN2022/092434
(87) International publication number: WO 2022/237868

(57) **Abstract**

A sample processing apparatus, comprising: a housing (101) having at least one chamber, and a valve body (301), the valve body (301) being coupled to the housing (101), the valve body (301) having a fluid displacement chamber (3012), a fluid port (3013), and a fluid channel (3014) fluidly connecting the fluid displacement chamber (3012) and the fluid port (3013), the fluid displacement chamber (3012) being coupled to a pressure adjustment member (1027), and the pressure adjustment member (1027) being configured to change a pressure within the fluid displacement chamber (3012); the valve body (301) being adjustable with respect to the housing (101), such that the fluid port (3013) of the valve body (301) is selectively in fluidic communication with the at least one chamber of the housing (101).

## Description

### TECHNICAL FIELD

The present disclosure relates to an integrated apparatus for sample loading, processing and reaction, a system for assaying the sample and a method for processing the sample.

### BACKGROUND OF THE INVENTION

Nucleic acid amplification methods permit selected amplification and identification of nucleic acids of interest from a complex mixture, such as a biological sample. To detect a nucleic acid in a biological sample, the biological sample is typically processed to isolate nucleic acids from other components of the biological sample and other agents that can interfere with the nucleic acid and/or amplification. Following isolation of the nucleic acid of interest from the biological sample, the nucleic acid of interest can be amplified, via, for example, amplification methods, such as thermal cycling based approaches (e.g., polymerase chain reaction (PCR)). Following amplification of the nucleic acid of interest, the products of amplification can be detected and the results of detection can be interpreted by an end-user.

### SUMMARY OF THE INVENTION

In an aspect, the present disclosure provides a sample processing apparatus. The sample processing apparatus can comprise a housing having at least one chamber, the at least one chamber being configured to contain therein at least one reagent for processing a biological sample; and a valve body coupled to the housing, the valve having a fluid displacement chamber, a fluid port and a fluid channel fluidically connecting the fluid displacement chamber and the fluid port, the fluid displacement chamber being coupled to a pressure adjustment member which is configured to change a pressure within the fluid displacement chamber. The valve body can be adjustable with respect to the housing, such that the fluid port of the valve body can be selectively in fluidic communication with the at least one chamber of the housing.

In another aspect, the present disclosure provides a sample processing method. The method can comprise providing the sample processing apparatus of the present disclosure; coupling the pressure adjustment member to the fluid displacement chamber, the pressure adjustment member being operated to change a pressure within the fluid displacement chamber; and adjusting a position of the valve body with respect to the housing, such that the fluid port of the valve body can be selectively in fluidic communication with the at least one chamber of the housing.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, of which:
FIG. 1 is a schematic depicting an exemplary sample processing apparatus of the present disclosure.
FIGs. 2A and 2B are schematics depicting a structure of an exemplary sample processing apparatus of the present disclosure.
FIG. 3 is a schematic depicting chambers of an exemplary sample processing apparatus of the present disclosure.
FIG. 4 is a schematic depicting a valve body of an exemplary sample processing apparatus of the present disclosure.
FIG. 5 is a cross-sectional view depicting a valve body of an exemplary sample processing apparatus of the present disclosure.
FIG. 6 depicts the operating principles of an exemplary sample processing apparatus of the present disclosure.
FIG. 7A depicts an arrangement of chambers within a housing of an exemplary sample processing apparatus of the present disclosure, and FIG. 7B depicts an bottom of a valve body of an exemplary sample processing apparatus of the present disclosure.
FIG. 8 depicts an arrangement of chambers within a housing of another exemplary sample processing apparatus of the present disclosure.
FIG. 9 depicts a structure of an exemplary sample processing apparatus of the present disclosure.
FIG. 10 is a cross-sectional view depicting an exemplary reaction vessel coupled with a sample processing apparatus of the present disclosure.
FIG. 11 is an enlarged view of part A in FIG. 10.
FIG. 12 depicts a cap configured to cover the reaction vessel.
FIG. 13 depicts a consumable operating mechanism for operating an exemplary sample processing apparatus of the present disclosure mated with a sample processing apparatus.
FIG. 14 shows a computer system programmed or otherwise configured to implement the sample processing method and/or detection method provided by the present disclosure.

### DETAILED DESCRIPTION OF THE INVENTION

While various embodiments of the present invention have been shown and described herein, it will be obvious to those skilled in the art that such embodiments are provided by way of example only. Numerous variations, changes, and substitutions can occur to those skilled in the art without departing from the invention. It should be understood that various alternatives to the embodiments of the invention described herein can be employed.

As used in the specification and claims, the singular form "a", "an", and "the" include plural references unless the context clearly dictates otherwise. For example, the term "a cell" includes a plurality of cells, including mixtures thereof.

As used herein, the terms "amplifying" and "amplification" generally refer to generating one or more copies of a nucleic acid or "amplified product". The term "DNA amplification" generally refers to generating one or more copies of a DNA molecule or "amplified DNA product". The term "reverse transcription amplification" generally refers to generating deoxyribonucleic acid (DNA) from a ribonucleic acid (RNA) template with a reverse transcriptase.

As used herein, the terms "denaturing" and "denaturation" generally refer to the full or partial unwinding of the helical structure of a double-stranded nucleic acid, and in some cases the unwinding of the secondary structure of a single-stranded nucleic acid. Denaturation can include the inactivation of the cell wall(s) of a pathogen or the shell of a virus, and the inactivation of the protein(s) of inhibitors. Conditions at which denaturation can occur include a "denaturation temperature" that generally refers to a temperature at which denaturation can occur and a "denaturation duration" that generally refers to an amount of time allotted for denaturation to occur.

As used herein, the term "elongation" generally refers to the incorporation of nucleotides to a nucleic acid in a template directed fashion. Elongation can occur via the aid of an enzyme, such as, for example, a polymerase or reverse transcriptase. Conditions at which elongation can occur include an "elongation temperature" that generally refers to a temperature at which elongation can occur and an "elongation duration" that generally refers to an amount of time allotted for elongation to occur.

As used herein, the term "nucleic acid" generally refers to a polymeric form of nucleotides of any length, either deoxyribonucleotides (dNTPs) or ribonucleotides (rNTPs), or analogs thereof. Nucleic acids can have any three-dimensional structure, and can perform any function, known or unknown. Non-limiting examples of nucleic acids include DNA, RNA, coding or non-coding regions of a gene or gene fragment, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant nucleic acids, branched nucleic acids, plasmids, vectors, isolated DNA of any sequence, isolated RNA of any sequence, nucleic acid probes, and primers. A nucleic acid can comprise one or more modified nucleotides, such as methylated nucleotides and nucleotide analogs. If present, modifications to the nucleotide structure can be made before or after assembly of the nucleic acid. The sequence of nucleotides of a nucleic acid can be interrupted by non-nucleotide components. A nucleic acid can be further modified after polymerization, such as by conjugation or binding with a reporter agent.

As used herein, the term "primer extension reaction" generally refers to the denaturing of a double-stranded nucleic acid, binding of a primer to one or both strands of the denatured nucleic acid, followed by elongation of the primer(s).

As used herein, the term "reaction mixture" generally refers to a composition comprising reagents used to complete nucleic acid amplification (e.g., DNA amplification, RNA amplification), with non-limiting examples of such reagents that include primer sets having specificity for target RNA or target DNA, DNA produced from reverse transcription of RNA, a DNA polymerase, a reverse transcriptase (e.g., for reverse transcription of RNA), suitable buffers (including zwitterionic buffers), co-factors (e.g., divalent and monovalent cations), dNTPs, and other enzymes (e.g., uracil-DNA glycosylase (UNG)), etc.). In some cases, reaction mixtures can also comprise one or more reporter agents.

As used herein, the term "target nucleic acid" generally refers to a nucleic acid molecule in a starting population of nucleic acid molecules having a nucleotide sequence whose presence, amount, and/or sequence, or changes in one or more of these, are desired to be determined. A target nucleic acid can be any type of nucleic acid, including DNA, RNA, and analogs thereof. As used herein, a "target ribonucleic acid (RNA)" generally refers to a target nucleic acid that is RNA. As used herein, a "target deoxyribonucleic acid (DNA)" generally refers to a target nucleic acid that is DNA.

As used herein, the term "subject," generally refers to an entity or a medium that has testable or detectable genetic information. A subject can be a person or individual. A subject can be a vertebrate, such as, for example, a mammal. Non-limiting examples of mammals include murines, simians, humans, farm animals, sport animals, and pets. Other examples of subjects include food, plant, soil, and water.

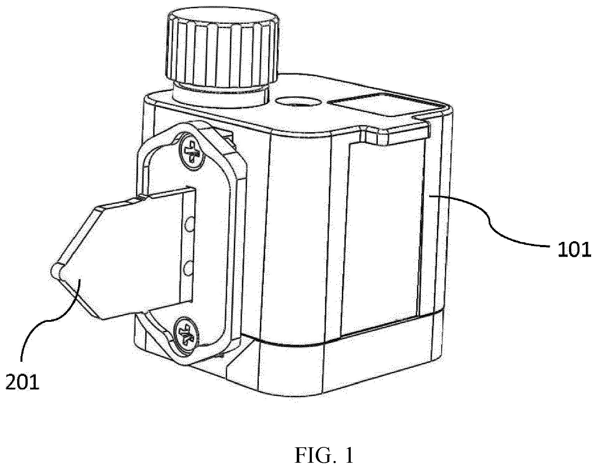

FIG. 1 depicts a sample processing apparatus according to an exemplary embodiment of the present disclosure. The sample processing apparatus can comprise a housing 101. The housing 101 can be configured to receive a biological sample, perform one or more processes on the biological sample and generate a reaction mixture. The reaction mixture can be transferred to a reaction vessel 201 fluidically coupled to the housing 101 and subjected to, for example, a nucleic acid amplification reaction in the reaction vessel. In some instances, the sample processing apparatus can be a consumable that operates with a thermal cycling apparatus (e.g., a PCR thermal cycler) to amplify nucleic acids of interest in the sample and detect an amplified product. For example, the reaction vessel 201 can be heated and cooled by the thermal cycling apparatus to subject the reaction mixture comprising the biological sample in the reaction vessel to a polymerase chain reaction on. In some instances, the reaction vessel can be provided with a thin shape. In an example, the reaction vessel can be dimensioned with a thickness suitable for insertion into the thermal cycling apparatus, such that the reaction vessel can be thermally coupled with a heater and/or a heat sink in the thermal cycling apparatus. The sample processing apparatus of the present disclosure can be closed. For example, once the biological sample is added into the sample processing apparatus, any processing and reactions of the biological sample can be performed within the sample processing apparatus.

A target nucleic acid can be amplified in the reaction vessel 201 to generate an amplification product. The target nucleic acid can be a target RNA or a target DNA. In some embodiments, the target RNA is viral RNA. In some embodiments, the viral RNA may be pathogenic to the subject. Non-limiting examples of pathogenic viral RNA include human immunodeficiency virus I (HIV I), human immunodeficiency virus II (HIV II), orthomyxoviruses, Ebola virus, Dengue virus, influenza viruses (e.g., H1N1, H3N2, H7N9, or H5N1), hepatitis virus, hepatitis A virus, hepatitis B virus, hepatitis C virus (e.g., RNA-HCV virus), hepatitis D virus, hepatitis E virus, hepatitis G virus, Epstein-Barr virus, mononucleosis virus, cytomegalovirus, SARS virus, West Nile Fever virus, polio virus, measles virus, or coronavirus (e.g., novel coronavirus COVID-19). In cases where the target nucleic acid is a target DNA, the target DNA may be any type of DNA, including types of DNA described elsewhere herein. In some embodiments, the target DNA is viral DNA. In some embodiments, the viral DNA may be pathogenic to the subject. Non-limiting examples of DNA viruses include herpes simplex virus, smallpox, adenovirus (e.g., Adenovirus Type 55, Adenovirus Type 7) and Varicella virus (e.g., chickenpox). In some cases, a target DNA may be a bacterial DNA. The bacterial DNA may be from a bacterium pathogenic to the subject such as, for example, *Mycobacterium tuberculosis* -a bacterium known to cause tuberculosis. In some cases, a target DNA may be a DNA from a pathogenic protozoan, such as, for example one or more protozoans of the *Plasmodium* type that can cause Malaria.

Any type of nucleic acid amplification reaction known in the art can be used to amplify a target nucleic acid and generate an amplified product. Moreover, amplification of a nucleic acid can linear, exponential, or a combination thereof. Amplification can be emulsion based or can be non-emulsion based. Non-limiting examples of nucleic acid amplification methods include reverse transcription, primer extension, polymerase chain reaction, ligase chain reaction, helicase-dependent amplification, asymmetric amplification, rolling circle amplification, and multiple displacement amplification (MDA). In some embodiments, the amplified product can be DNA. In cases where a target RNA is amplified, DNA can be obtained by reverse transcription of the RNA and subsequent amplification of the DNA can be used to generate an amplified DNA product. The amplified DNA product can be indicative of the presence of the target RNA in the biological sample. In cases where DNA is amplified, any DNA amplification method known in the art can be employed. Non-limiting examples of DNA amplification methods include polymerase chain reaction (PCR), variants of PCR (e.g., real-time PCR, allele-specific PCR, assembly PCR, asymmetric PCR, digital PCR, emulsion PCR, dial-out PCR, helicase-dependent PCR, nested PCR, hot start PCR, inverse PCR, methylation-specific PCR, miniprimer PCR, multiplex PCR, nested PCR, overlap-extension PCR, thermal asymmetric interlaced PCR, touchdown PCR), and ligase chain reaction (LCR). In some cases, DNA amplification is linear. In some cases, DNA amplification is exponential. In some cases, DNA amplification is achieved with nested PCR, which can improve sensitivity of detecting amplified DNA products.

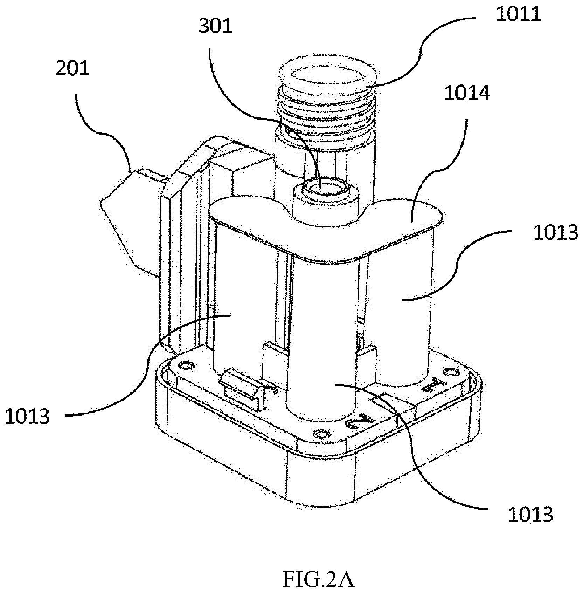

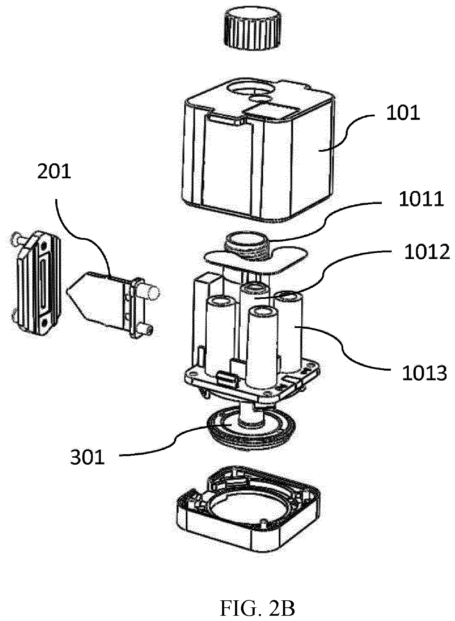

Referring to FIGs. 2A and 2B in which the enclosure of the sample processing apparatus is removed to show the internal configuration, the housing 101 can enclosed therein at least one chamber. In an exemplary example, the at least one chamber can comprise a sample chamber 1011 configured to receive therein a biological sample. The sample chamber 1011 can be provided with a removable lid (e.g., a screw lid, a snap lid, or a flip lid) for sealing an opening of the sample chamber after the biological sample is added. In some instances, reagents necessary for performing a nucleic acid amplification (e.g., DNA amplification, RNA amplification) can be pre-stored in the sample chamber.

Any suitable biological sample that comprises nucleic acid can be obtained from a subject. A biological sample can be a solid matter (e.g., a biological tissue) or can be a fluid (e.g., a biological fluid). In general, a biological fluid can include any fluid associated with living organisms. Non-limiting examples of a biological sample include blood (or components of blood - e.g., white blood cells, red blood cells, platelets) obtained from any anatomical location (e.g., tissue, circulatory system, bone marrow) of a subject, cells obtained from any anatomical location of a subject, skin, heart, lung, kidney, breath, bone marrow, stool, semen, vaginal fluid, interstitial fluids derived from tumorous tissue, breast, pancreas, cerebral spinal fluid, tissue, throat swab, biopsy, placental fluid, amniotic fluid, liver, muscle, smooth muscle, bladder, gall bladder, colon, intestine, brain, cavity fluids, sputum, pus, microbiota, meconium, breast milk, prostate, esophagus, thyroid, serum, saliva, urine, gastric and digestive fluid, tears, ocular fluids, sweat, mucus, earwax, oil, glandular secretions, spinal fluid, hair, fingernails, skin cells, plasma, nasal swab or nasopharyngeal wash, spinal fluid, cord blood, and/or other excretions or body tissues.

A biological sample can be obtained from a subject using various approaches known in the art. Non-limiting examples of approaches to obtain a biological sample directly from a subject include accessing the circulatory system (e.g., intravenously or intra-arterially via a syringe or other needle), collecting a secreted biological sample (e.g., feces, urine, sputum, saliva, etc. ), surgically (e.g., biopsy), swabbing (e.g., buccal swab, oropharyngeal swab), pipetting, and breathing. Moreover, a biological sample can be obtained from any anatomical part of a subject where a desired biological sample is located. In an exemplary embodiment, a subject's saliva comprising nucleic acids can be collected from the subject's oral cavity using a buccal swab, which is subsequently placed in a sample chamber. In another exemplary example, a syringe can be used to obtain a subject's blood from a subject's vein and the blood is added to a sample chamber. In some cases, the biological sample is obtained directly from a subject. A biological sample obtained directly from a subject generally refers to a biological sample that has not been further processed after being obtained from the subject, with the exception of any approaches used to collect the biological sample from the subject for further processing. For example, blood is obtained directly from a subject by accessing the subject's circulatory system, removing the blood from the subject (e.g., via a needle), and entering the removed blood into a receptacle. The receptacle can comprise reagents (e.g., anticoagulants) such that the blood sample is useful for further analysis. The blood can also be directly dropped into the sample chamber of the sample processing apparatus of the disclosure. In another example, a swab can be used to access epithelial cells on an oropharyngeal surface of the subject. After obtaining the biological sample from the subject, the swab containing the biological sample can be contacted with a fluid (e.g., a buffer) to collect a biological fluid from the swab, or the swab containing the biological sample can be placed directly in the sample chamber of the sample processing apparatus of the disclosure. In some embodiments, a biological sample has not been purified when provided in a reaction vessel. In some embodiments, the nucleic acid of a biological sample has not been extracted when the biological sample is provided to the sample chamber. For example, the RNA or DNA in a biological sample may not be extracted from the biological sample when providing the biological sample to the sample chamber of the sample processing apparatus of the disclosure. Moreover, in some embodiments, a target nucleic acid (e. g., a target RNA or target DNA) present in a biological sample may not be concentrated prior to providing the biological sample to the sample chamber of the sample processing apparatus of the disclosure.

In some embodiments, the housing of the sample processing apparatus can have only one chamber, i.e., the sample chamber. All reagents necessary for preforming the sample processing and nucleic acid amplification (e.g., DNA amplification, RNA amplification) can be pre-stored in the sample chamber. Reagents necessary for processing the biological sample can include reagents that are necessary for reverse transcription and nucleic acid amplification (e.g., reverse transcriptase, DNA polymerase, dNTPs, co-factors, primers, suitable buffer, etc.) and reporter agents (e.g., an oligonucleotide probe comprising FAM dye). Once the biological sample of the subject is added, any processing of the sample required for nucleic acid amplification can be performed within the sample chamber.

In other embodiments, in addition to the sample chamber, the housing of the sample processing apparatus can have at least one reagent chamber 1013 configured to contain therein one or more reagents necessary for processing the biological sample. The number of reagent chambers can be determined based on a type of sample, type of subsequent nucleic acid amplification, etc. The number of reagent chambers can be 1, 2, 3, 4, 5, 6, 7, 8, 9, 10 or more. At least one reagent chamber can be provided on the same base as the sample chamber with their extending axes being substantially parallel with each other. Each of the at least one reagent chambers can have an opening end, which opening end can be provided in the same direction as the opening end of the sample chamber. A sealing element 1014 can be provided to the at least one reagent chamber to seal the opening end of thereof. In some instances, a separate sealing element can be provided to each reagent chamber. In some instances, a single sealing element can be provided to seal all the opening ends of the reagent chambers. The sealing element can be made of elastic material.

The sealing element can enable a pressure change in the reagent chamber within a certain range without rupture. In some instances, the pressure within the reagent chamber can be atmospheric pressure. In some instances, the pressure within the reagent chamber can be at least 1.1 times, 1.2 times, 1.3 times, 1.4 times, 1.5 times, 1.6 times, 1.7 times, 1.8 times, 1.9 times, 2.0 times, 2.1 times, 2.2 times, 2.3 times, 2.4 times, 2.5 times, 2.6 times, 2.7 times, 2.8 times, 2.9 times, 3.0 times, 3.5 times, 4.0 times, 4.5 times or 5.0 times of the atmospheric pressure. In some instances, the pressure within the reagent chamber can be at least 0.9 times, 0.8 times, 0.7 times, 0.6 times, 0.5 times, 0.4 times, 0.3 times, 0.2 times, 0.1 times, 0.09 times, 0.08 times, 0.07 times, 0.06 times, 0.05 times, 0.04 times, 0.03 times, 0.02 times, 0.01 times, 0.005 times or 0.001 times the atmospheric pressure. The pressure within the reagent chamber can be any value between any two of the aforesaid numeric values.

Each reagent chamber can be pre-stored therein one or more reagents necessary for processing the biological sample. In an exemplary embodiment, in addition to the sample chamber, three reagent chambers can be provided within the housing of the sample processing apparatus. The sample chamber can be pre-stored with a sample preservation solution. A sampling swab can be directly placed into the sample chamber and contacted with the sample preservation solution. The first reagent chamber can be a processing solution chamber in which a sample processing solution is prefilled. The sample processing solution and the biological sample can be mixed in preset proportion to obtain a processed sample. The second reagent chamber can be a solid reagent chamber in which a solid reagent (e.g., lyophilized powder, reagent pellets, etc.) for preparing a PCR reaction solution is preloaded. The solid reagent, which is used to prepare the PCR reaction solution, can comprise, for example, components such as RT enzyme, taq enzyme and primer probe. The third reagent chamber can be a reconstitution buffer chamber in which a reconstitution buffer for PCR reaction solution lyophilized powder is prefilled. Once the PCR reaction solution lyophilized powder in the second reagent chamber is dissolved with the reconstitution buffer, it can be mixed with the sample that is processed with the sample processing solution in the first reagent chamber to obtain a reaction mixture for performing a PCR amplification reaction.

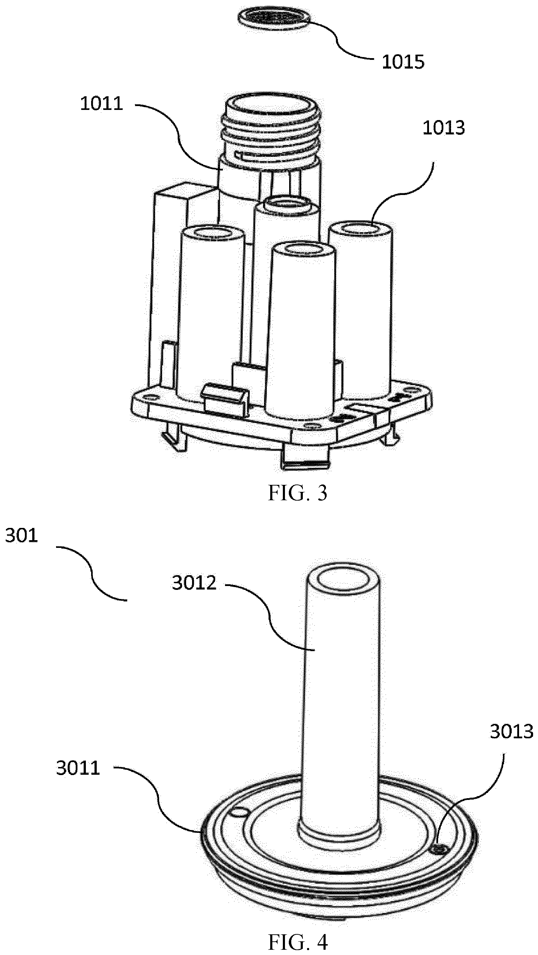

FIG. 3 is another schematic depicting the chamber structure of an exemplary sample processing apparatus. As shown in FIG. 3, the housing of the sample processing apparatus can enclose therein a sample chamber 1011 and optionally one or more reagent chambers 1013. The housing can further comprise a reaction vessel interface 1016 configured to couple with the reaction vessel, such that the reaction mixture can be transported into the reaction vessel for nucleic acid amplification reaction. The reaction vessel interface 1016 can comprise a reaction vessel fluid inlet interface and a reaction vessel fluid outlet interface that are coupled to a reaction vessel fluid inlet and a reaction vessel fluid outlet of the reaction vessel, respectively. The reaction vessel fluid inlet interface of the reaction vessel interface can be coupled with a reaction mixture channel in the sample processing apparatus. The reaction vessel fluid inlet interface of the reaction vessel interface can be configured to transport the reaction mixture to the reaction vessel for, for example, an amplification reaction. The sample chamber 1011 can be provided with a filter 1015 which is configured to filter out impurities in the biological sample. The filter can be flexible or rigid. For example, in situation where a cotton swab is used to collect a subject's saliva and the swab is then dropped into the sample chamber, the filter can prevent any cotton fiber from entering a subsequent sample processing. A pore size of a mesh of the filter can vary depending on the type of biological sample collected, the tool used to collect the biological sample, the processing performed on the sample, etc. The housing of the sample processing apparatus can further comprise a bottom seal. The bottom seal can be provided with a disc shape and configured to seal a bottom opening of the sample chamber and bottom openings of the one or more reagent chambers. The bottom seal can be made of an elastic material, such as rubber or silicone rubber. In some instances, a through hole corresponding to the sample chamber and the reagent chamber(s) can be provided on the bottom seal, such that fluid can enter or leave the sample chamber and the reagent chamber(s) via the through holes.

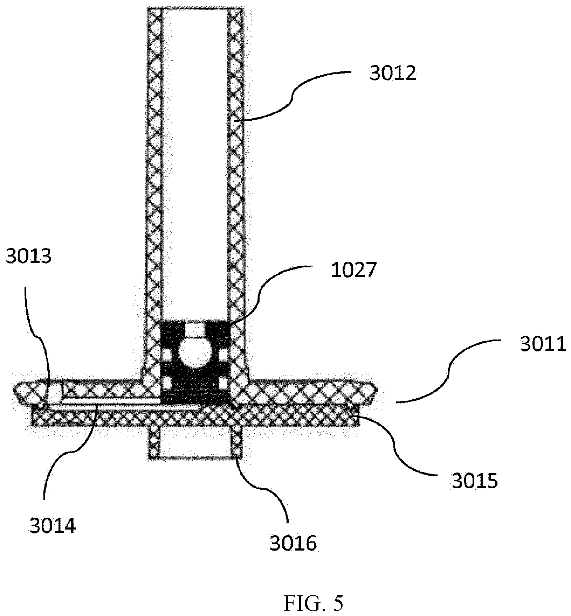

The sample processing apparatus of the present disclosure can further comprise a valve body 301 coupled to the housing 101, as shown in FIGs. 2A and 2B. FIG. 4 depicts an exemplary structure of the valve body. FIG. 5 depicts a cross-sectional view of the valve body of an exemplary sample processing apparatus of the present disclosure. The valve body 301 can include a base portion 3011 and a fluid displacement chamber 3012 which is in communication with the base portion. In an embodiment, the fluid displacement chamber of the valve body can be inserted into the housing via a through hole in the bottom seal of the housing of the sample processing apparatus to finish an assembly of the housing and the valve body. Once assembled, a longitudinal axis of the fluid displacement chamber of the valve body can be substantially parallel to a longitudinal axis of the sample chamber and a longitudinal axis of the reagent chamber of the housing. The base portion of the valve body can be positioned below the housing and configured to firmly fit (e.g., in a liquid-tight manner) with the bottom seal of the housing, as shown in FIGs. 2A and 2B.

The base portion of the valve body can be provided with a disk shape. The fluid displacement chamber of the valve body can be provided with a cylindrical shape. The fluid displacement chamber of the valve body can comprise a chamber with a predetermined volume. A cross-section of the fluid displacement chamber can be circular, oval, rectangular, square, triangular, etc. A distal end of the fluid displacement chamber (i.e., the end away from the base portion of the valve body) can be open, while a proximal end of the fluid displacement chamber (i.e., the end coupled to the base portion of the valve body) can be in fluidic communication with a fluid channel in the base portion. The fluid channel can be formed using a thermal welding or thermal bonding process. A cross-sectional area of the fluid channel can enable a fluid transfer. A fluid port 3013 can be provided on the base portion of the valve body. A fluid channel 3014 can be provided in the base portion to fluidically communicate an internal volume of the fluid displacement chamber 3012 with the fluid port 3013. In some instances, the fluid port can be provided at a position in proximity to a radial edge of a side of the base facing the housing of the sample processing apparatus. Although only one fluid port and one fluid channel are shown in the embodiment of FIGs. 4 and 5, multiple fluid ports and multiple fluid channels can be provided to the base portion of the valve body, each of the multiple fluid channel fluidically communicating an internal volume of the fluid displacement chamber with a corresponding fluid port.

In some instances, the base portion of the valve body can comprise a base engagement 3015 for coupling with a consumable operating mechanism for operating the sample processing apparatus of the present disclosure. In some instances, a valve mating portion 3016 can be provided on the base engagement. The valve mating portion can comprise, for example, a groove or a protrusion which is configured to engage with a corresponding protrusion or groove of the consumable operating mechanism, thereby adjusting (e.g., rotating) the valve body with respect with the housing by the consumable operating mechanism.

The fluid displacement chamber of the valve body can be coupled with a pressure adjustment member 1027. The pressure adjustment member can be configured to change the pressure within the fluid displacement chamber. As shown in in FIG. 5 which is the cross-sectional view of the valve body of the sample processing apparatus, the pressure adjustment member can be a plunger or a piston that firmly fits with an inner wall of the fluid displacement chamber. Once the sample processing apparatus of the present disclosure is coupled to the consumable operating mechanism, the pressure adjustment member can be coupled to a shaft of the consumable operating mechanism, enabling a movement of the pressure adjustment member in the fluid displacement chamber relative to the base portion of the valve body (for example, in an up and down direction in FIG. 5) under the action of the shaft of the consumable operating mechanism. One or more displacement limiting members can be provided in the fluid displacement chamber to limit the displacement of the pressure adjustment member. In some instances, one or more protrusions, flange rings, steps or diameter reducing parts can be provided on the inner wall of the fluid displacement chamber to prevent a further movement of the pressure adjustment member when the pressure adjustment member engages the displacement limiting member. In some instances, the pressure adjustment member can move across the one or more displacement limiting members within the fluid displacement chamber. Once the pressure adjustment member moves across the displacement limiting member, a signal can be generated to indicate an amount of displacement of the pressure adjustment member within the fluid displacement chamber. In some instances, the pressure adjustment member cannot move cross at least one of the displacement limiting members, such that a further movement of the pressure adjustment member within the fluid displacement chamber is blocked by the displacement limiting members.

A change in the pressure within the fluid displacement chamber can be generated by a linear movement of the pressure adjustment member within the fluid displacement chamber. A decrease in pressure within the fluid displacement chamber can draw fluid into the fluid displacement chamber via the fluid port 3013 and the fluid channel 3014. An increase in pressure within the fluid displacement chamber can expel fluid from the fluid displacement chamber via the fluid channel 3014 and the fluid port 3013. One or more displacement limiting members in the fluid displacement chamber can be used to control or indicate the amount of pressure change in the fluid displacement chamber. In some instances, the pressure adjustment member can be a part of the sample processing apparatus of the present disclosure. For example, a plunger or piston can be provided in the fluid displacement chamber of the valve body as part of the sample processing apparatus being manufactured. In another example, the pressure adjustment member may not be part of the sample processing apparatus of the present disclosure. In this case, the pressure adjustment member can be provided by the consumable operating mechanism and placed into the fluid displacement chamber of the valve body when the sample processing apparatus of the present disclosure is coupled with the consumable operating mechanism.

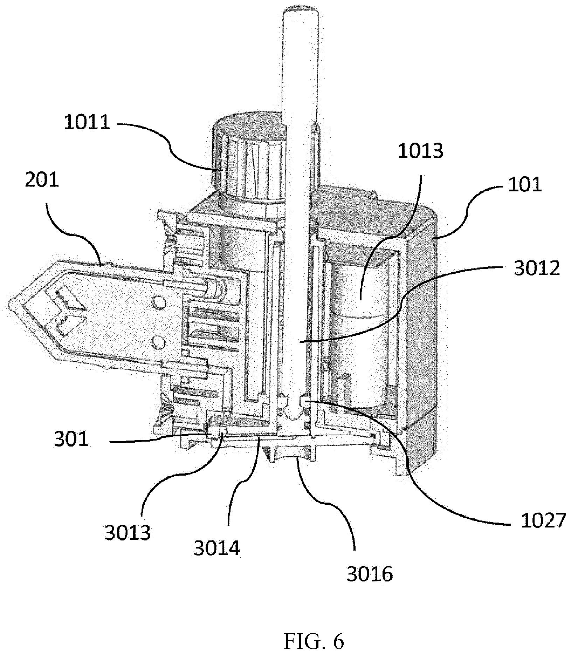

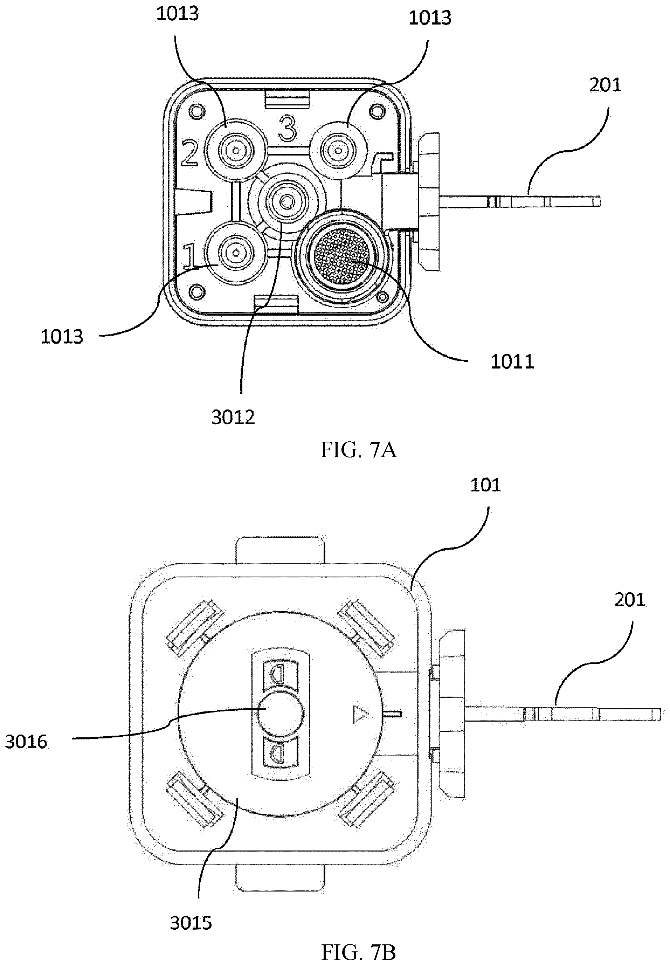

FIG. 6 is a schematic depicting the operating principles of an exemplary sample processing apparatus. The base portion 3011 of the valve body 301 can be positioned below the housing 101. The fluid displacement chamber 3012 of the valve body can be inserted into the housing from the bottom of the housing (e.g., via a through hole in the bottom seal of the housing). The position at which the fluid displacement chamber of the valve body is inserted into the housing can be near or offset a geometric center of the bottom of the housing. Once the fluid displacement chamber of the valve body is inserted into the housing, a longitudinal axis of the fluid displacement chamber can be substantially parallel to longitudinal axes of the sample chamber 1011 and the one or more reagent chambers 1013. FIG. 7A depicts an arrangement and relative position of chambers within the housing and the fluid displacement chamber of the valve body in an exemplary sample processing apparatus. In some instances, the fluid displacement chamber 3012 of the valve body can be surrounded by the sample chamber 1011 and one or more reagent chambers 1013. In a direction perpendicular to the longitudinal axis of the fluid displacement chamber of the valve body (as shown in FIG. 7A), the longitudinal axis of the fluid displacement chamber of the valve body can be positioned at the geometric center of a geometric shape enclosed by the longitudinal axes of the sample chamber and the one or more reagent chambers. In the example shown in FIG. 7A, the longitudinal axes of the sample chamber and the reagent chambers can form a substantially square or circle in a top view, and the fluid displacement chamber of the valve body can be positioned with a longitudinal axis there at the geometric center of the substantially square or circle.

The valve body can be rotatably adjustable around the longitudinal axis thereof relative to the housing of the sample processing apparatus. In some embodiments, once the sample processing apparatus is mated with the consumable operating mechanism, the valve mating portion 3016 (for example, a groove or a protrusion) provided on the base portion of the valve body can be engaged with a corresponding protrusion or groove of the consumable operating mechanism, enabling a rotation of the valve body with respect to the housing of the sample processing apparatus under a driving of the consumable operating mechanism. The relative rotation of the valve body with respect to the housing can place the fluid port 3013 of the base portion of the valve body selectively in fluidic communication with a bottom opening of the sample chamber 1011, a bottom opening of one of the one or more reagent chambers 1013, or an fluid inlet of the reaction vessel 201, such that an internal volume of the fluid displacement chamber 3012 of the valve body is selectively in fluidic communication with an internal volume of the sample chamber 1011, an internal volume of one of one or more reagent chambers 1013, and/or an fluid inlet of the reaction vessel 201 through the fluid channel 3014 and fluid port 3013 of the base portion of the valve body. Although only one fluid port and one fluid channel are illustrated in FIG. 6, multiple fluid ports and multiple fluid channels can be provided on the base portion of the valve body, each of the multiple fluid channels fluidically communicating the internal volume of the fluid displacement chamber with a corresponding fluid port. In this way, the internal volume of the fluid displacement chamber of the valve body can be simultaneously in fluidic communication with the internal volume of the sample chamber 1011 and the internal volume of one or more of the one or more reagent chambers 1013. In the embodiments where multiple fluid ports and multiple fluid channels are provided at the base portion of the valve body, the number of the internal volume of the sample chamber and the internal volume of the one or more reagent chambers being in fluidic communication with the internal volume of the fluid displacement chamber of the valve body simultaneously can be controlled by changing a position and arrangement of the multiple fluid ports on the base portion of the valve body. For example, in an embodiment where the housing has one sample chamber and three reagent chambers, by controlling ae rotational position of the valve body with respect to the housing, the internal volume of the fluid displacement chamber of the valve body can be selectively in fluidic communication with, at a particular timing, (i) only the internal volume of the sample chamber, (ii) only one of the internal volumes of the three reagent chambers, (iii) two of the internal volumes of the three reagent chambers, (iv) all the internal volumes of the three reagent chambers, (v) the internal volume of the sample chamber and one of the internal volumes of the three reagent chambers, (vi) the internal volume of the sample chamber and two of the internal volumes of the three reagent chambers, or (vii) the internal volume of the sample chamber and all the internal volumes of the three reagent chambers.

The pressure adjustment member 1027 can be at least partially positioned within the fluid displacement chamber of the valve body. The pressure adjustment member can comprise a plunger or piston firmly fitting with the inner wall of the fluid displacement chamber of the valve body and a connecting rod coupled to the plunger or piston. The plunger or piston and/or the connecting rod can be a component of the sample processing apparatus of the present disclosure. Alternatively, the plunger or piston and/or the connecting rod can be a component of the consumable operating mechanism that operates the sample processing apparatus of the present disclosure. A movement of the pressure adjustment member in the fluid displacement chamber of the valve body can change the pressure within the fluid displacement chamber. A decrease in pressure within the fluid displacement chamber can draw the fluid from the sample chamber and/or reagent chamber(s) into the volume of the fluid displacement chamber via the bottom opening(s) of the sample chamber and/or reagent chamber, the fluid port 3013 at the base portion of the valve body and the fluid channel 3014 in the base portion of the valve body. An increase in pressure within the fluid displacement chamber can drive fluid out from the volume of the fluid displacement chamber into the sample chamber, the reagent chamber(s) and/or the reaction vessel via the fluid channel 3014 in the base portion of the valve body, the fluid port 3013 at the base portion of the valve body and the bottom opening(s) of the sample chamber and/or reagent chamber. The amount of fluid drawn or expelled can be adjusted by controlling the displacement of the pressure adjustment member in the fluid displacement chamber of the valve body. As discussed hereinabove, the number of the internal volume of the sample chamber and the internal volume of the one or more reagent chambers being in fluidic communication with the internal volume of the fluid displacement chamber of the valve body simultaneously can be controlled by changing a position and arrangement of the multiple fluid ports on the base portion of the valve body. For example, by rotating the base portion of the valve body relative to the housing, the fluid displacement chamber of the valve body can be selectively in fluidic communication with one or more of the sample chamber, the one or more reagent chambers and/or the fluid inlet of the reaction vessel. Subsequently, by manipulating a direction and amount of displacement of the pressure adjustment member in the fluid displacement chamber of the valve body, a predetermined amount of fluid can be drawn from the sample chamber and/or the reagent chamber into the fluid displacement chamber of the valve body, or a predetermined amount of fluid can be driven from the fluid displacement chamber of the valve body into the selected sample chamber, reagent chamber and/or reaction vessel.

The rotational position of the valve body's base relative to the housing can be visually indicated by an indicator on the valve body's base engagement 3015. In the example shown in FIG. 7B, an arrow symbol on a bottom surface of the base engagement 3015 of the valve body can correspond to the position of the fluid port 3013 at the base portion 3011 of the valve body (e.g., in FIG. 7B, the fluid port 3013 and the arrow symbol can be positioned on opposite sides of the base portion 3011 of the valve body). In this way, during a rotation of the valve body relative to the housing, a visual indication can be provided to indicate a position of the fluid port and which one of the sample chamber, the one or more reagent chambers and/or the fluid inlet of the reaction vessel is currently in fluidic communication with the fluid port. As shown in FIG. 7B, the fluid port on the base portion of the valve body can be in fluidic communication with the fluid inlet of the reaction vessel. The pressure adjustment member can be operated (for example, pressing he pressure adjustment member downward in the fluid displacement chamber of the valve body) to increase the pressure in the fluid displacement chamber of the valve body, resulting in at least a portion of the fluid in the fluid displacement chamber of the valve body being driven into the reaction vessel via the fluid channel in the base of the body, the fluid port on the base portion of the valve body and the fluid inlet of the reaction vessel. Starting from the position shown in FIG. 7B, the fluid displacement chamber of the valve body can be selectively in fluidic communication with the sample chamber or the one or more reagent chambers by rotating the valve body clockwise or counterclockwise. In an initial state, the fluid displacement chamber of the valve body of the sample processing apparatus of the present disclosure can be fluidically isolated from the sample chamber or any reagent chamber in housing of the sample processing apparatus. For example, in an initial state, the fluid port on the base portion of the valve body of the sample processing apparatus can be offset from the bottom openings of any chambers in the housing of the sample processing apparatus.

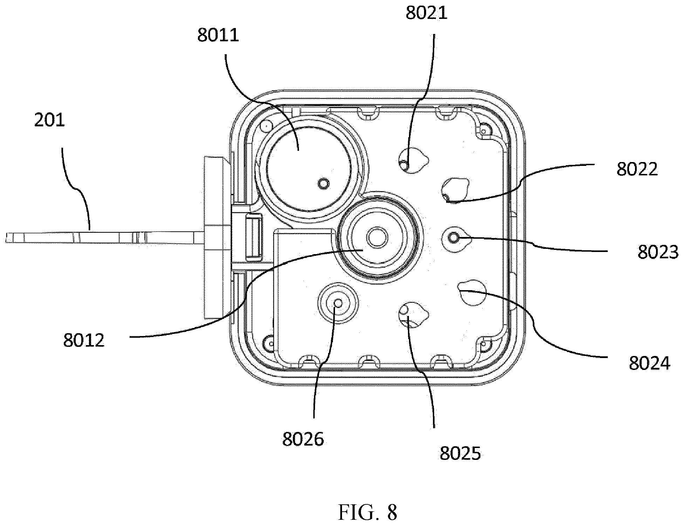

FIG. 8 is a view depicting an arrangement of chambers within a housing of another exemplary sample processing apparatus of the present disclosure. The fluid displacement chamber 8012 of a valve body can be surrounded by a sample chamber 8011 and one or more reagent chambers. In a direction perpendicular to the longitudinal axis of the fluid displacement chamber of the valve body, the longitudinal axis of the fluid displacement chamber of the valve body can be positioned at the geometric center (e.g., the center point) of a geometry (e.g., a circle) enclosed by the longitudinal axes of the sample chamber and the one or more reagent chambers. In some instances, multiple reagent chambers can comprise a chamber selected from a rinsing solution chamber 8022, a reconstitution buffer chamber 8023, an anti-adherence rinsing solution chamber 8024, a nucleic acid extraction chamber 8025 and a lyophilized pellet chamber 8026. In some embodiments, the housing of the sample processing apparatus can further comprise one or more non-reagent chambers, such as one or more waste liquid chambers 8021 and/or reserved chambers.

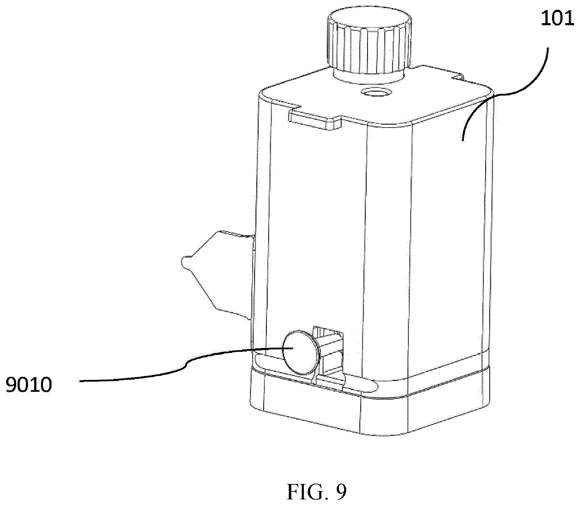

FIG. 9 depicts a structure of an exemplary sample processing apparatus of the present disclosure, in which magnetic beads can be employed in extracting nucleic acid from a biological sample. In some embodiments, magnetic beads can be pre-stored in the nucleic acid extraction chamber. Magnetic beads can be configured to adsorb nucleic acids in high-salt, low-PH solutions and release nucleic acids from the surface of magnetic beads in low-salt solutions. In this way, magnetic beads can be employed to extract nucleic acids from the biological sample. The magnetic beads can be nanomagnetic beads, for example silicone hydroxyl magnetic beads or carboxyl magnetic beads. In some instances, the magnetic beads can be spherical particles comprising magnetic microspheres of ferrosoferric oxide or ferric oxide and various materials such as silica containing active functional groups. The magnetic beads can be configured with instantaneous magnetic responsiveness, i.e., superparamagnetism. Therefore, the magnetic beads can be positioned, guided and separated under an external magnetic field.

As shown in FIG. 9, the housing 101 of the sample processing apparatus can be provided with an opening, through which a magnetic conductive component 9010 can be magnetically coupled to the nucleic acid extraction chamber, thereby coupling an external magnetic field with the nucleic acid extraction chamber. The external magnetic field can be adjusted to manipulate the magnetic beads within the nucleic acid extraction chamber. The magnetic conductive component 9010 can be made of a material having a high magnetic permeability, such as iron, nickel, etc.

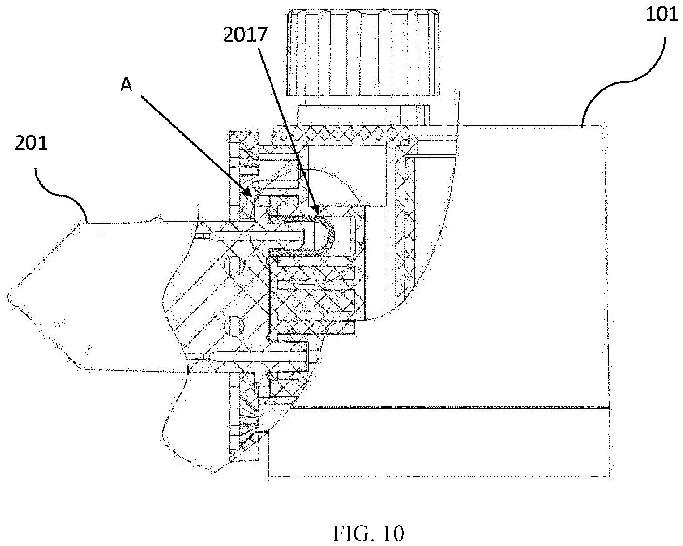

FIG. 10 is a cross-sectional view of an exemplary reaction vessel coupled with a sample processing apparatus. The sample processing apparatus can comprise a housing 101 and a valve body. The housing can have a sample chamber and optionally one or more reagent chambers. The valve body can comprise a base portion and a fluid displacement chamber which is in communication with the base portion. The fluid displacement chamber of the valve body can be inserted into the housing through a through hole at a bottom of the housing of the sample processing apparatus to mate the valve body with the housing. Under the control of the consumable operating mechanism, the valve body can be rotatably adjustable relative to the housing to selectively coupling the fluid displacement chamber of the valve body with the sample chamber or reagent chamber(s) of the housing via a fluid channel and a fluid port of the base portion of the valve body. The housing can comprise a reaction mixture channel having a first end (e.g., the upper end) coupled to a reaction vessel fluid inlet of the reaction vessel and a second end (e.g., the lower end) selectively coupled to the fluid port of the base portion of the valve body. In the status shown in FIG. 10, the fluid displacement chamber 3012 of the valve body can be in fluidic communication with the second one end (e.g., the lower end) of the reaction mixture channel in the housing via the fluid channel and the fluid port of the base portion of the valve body. As shown in FIG. 10, the reaction vessel 201 can be fluidically coupled with the housing 101. For example, the reaction vessel 201 can be inserted into the reaction vessel interface of the housing, enabling the reaction vessel fluid inlet of the reaction vessel 201 fluidically coupled with the first end (e.g., the upper end) of the reaction mixture channel of the housing.

The pressure adjustment member such as a plunger can be provided in the fluid displacement chamber of the valve body. The pressure within the fluid displacement chamber can be changed by operating the pressure adjustment member, thereby driving liquid into or expelling liquid from the fluid displacement chamber. In the status shown in FIG. 10, the pressure in the fluid displacement chamber of the valve body can be increased by operating the pressure adjustment member (for example, pressing the plunger down toward the base portion of the valve body) to drive the fluid (e.g., reaction mixture) toward the reaction vessel fluid inlet of the reaction vessel via the fluid channel and the fluid port of the base portion of the valve body. As the pressure in the fluid displacement chamber of the valve body being further increased, the reaction mixture be driven into the reaction region in the reaction vessel via the reaction vessel fluid inlet of the reaction vessel.

Exemplary reaction vessels of the present disclosure can be provided with a thin plate shape. The reaction vessel can be dimensioned and shaped to be suitable for mating with a thermal cycling apparatus for nucleic acid amplification and/or a detection apparatus for detecting a reaction product. In some instances, the reaction vessel can be dimensioned and shaped to be suitable for insertion into a slot provided on the thermal cycling apparatus and/or detection apparatus. In some instances, the reaction vessel can comprise a frame and two walls sandwiching the frame. The frame can be a rigid structure having a plurality of brackets. The two walls can be made of an elastic material. The two walls can be sheets or films which are attached to the frame, thereby forming within the frame at least one reaction region and a reaction vessel fluid input channel and a reaction vessel fluid output channel in fluidic communication with the reaction region. In some instances, the two walls can contact a heating member and/or cooling member in the thermal cycling apparatus, thereby efficiently heating and/or cooling the reaction mixture in the reaction region. The reaction vessel fluid input channel and the reaction vessel fluid output channel can be respectively in fluidic communication with the reaction vessel fluid inlet and the reaction vessel fluid outlet. A fluid from a chamber in the housing of the sample processing apparatus or the fluid displacement chamber of the valve body can be driven into the reaction vessel via the reaction vessel fluid inlet of the reaction vessel, subsequently into the reaction region via the reaction vessel fluid input channel and subjected to an amplification reaction in the reaction region. Fluid, including gas and liquid, can be expelled from the reaction region of the reaction vessel via the reaction vessel fluid output channel and the reaction vessel fluid outlet.

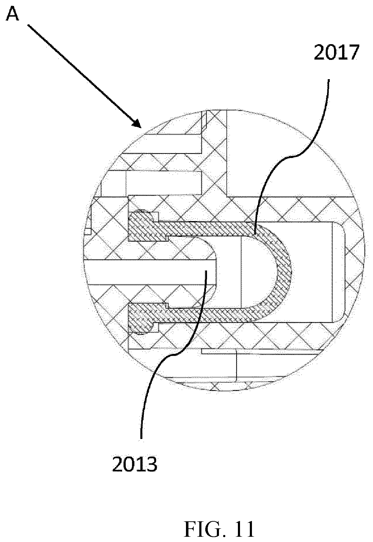

In an exemplary embodiment as shown in FIG. 11, the reaction vessel fluid outlet 2013 of a reaction vessel can be provided with an elastic seal 2017. The elastic seal can be configured to seal the reaction vessel fluid outlet of the reaction vessel in an air-tight and liquid-tight manner. The elastic seal can be made of an elastic material. When the reaction mixture enters the reaction vessel from the fluid displacement chamber of the valve body via the reaction vessel fluid inlet of the reaction vessel under an increased pressure generated by the pressure adjustment member, the air existing in the reaction vessel fluid input channel, the reaction region and the reaction vessel fluid output channel can be driven into the elastic seal 2017 via the reaction vessel fluid outlet 2013. As the pressure generated by the pressure adjustment member further increases, a portion of the reaction mixture can also be driven into the elastic seal 2017. A volume of the elastic seal can be increased due to the air and/or reaction mixture entering the elastic seal. Due to the elasticity of the elastic material, the increased volume of the elastic seal can generate a reverse pressure that exerts to the reaction region via the reaction vessel fluid outlet 2013 and the reaction vessel fluid output channel. Under the reverse pressure, the two walls of the reaction region can bulge outward, thereby further fitting the two walls of the reaction region onto the heating and/or cooling member of the thermal cycling apparatus. In this way, an efficient heating and cooling of the reaction mixture in the reaction region can be achieved which accelerates the amplification reaction. The elastic seal can be configured with various internal volumes. In some instances, the internal volume of the elastic seal can be determined based on at least one of a dimension of the reaction vessel, a dimension of the reaction vessel fluid input channel, a dimension of the reaction region, a dimension of the reaction vessel fluid output channel, a type of reaction occurring in the reaction vessel, and an amount of the reaction mixture. The internal volume of the elastic seal can be determined such that during a reaction process, the two walls of the reaction region bulge outward under the reverse pressure generated by the elastic seal and fit firmly onto the heating and/or cooling member of the thermal cycling apparatus.

Referring to FIG. 11 which is an enlarged view of part A in FIG. 10, in the process the reaction mixture entering the reaction vessel from the fluid displacement chamber of the valve body via the fluid channel of the base portion of the valve body, the air originally existing in the reaction mixture channel 3017, the reaction vessel fluid input channel 2014, the reaction region and the reaction vessel fluid output channel 2015 can be driven into the elastic seal 2017 that seals the reaction vessel fluid outlet 2013 of the reaction vessel 201. The elastic seal expands with the air, generating a reverse pressure that acts onto the reaction region. Under the action of the reverse pressure, the two walls of the reaction region can be moved outwards, thereby further fitting the two walls of the reaction region onto the heating and/or cooling member of the thermal cycling apparatus. In this way, an efficient heating and cooling of the reaction mixture in the reaction region can be achieved which accelerates the amplification reaction. The elastic seal can be provided as a balloon. A base portion of the elastic seal can be secured into a groove that is provided near the reaction vessel fluid outlet 2013 of the reaction vessel 201. When the reaction vessel is coupled to the housing of the sample processing apparatus (for example, at the reaction vessel interface 1016 shown in FIG. 3), a gap can be formed between the groove near the reaction vessel fluid outlet of the reaction vessel and the housing of the sample processing apparatus. The base portion of the elastic seal can be accommodated and secured in the gap, as shown in FIG. 11.

An elastic sealing member (such as a gasket) can be provided between the reaction vessel interface 1016 of the housing 101 and the reaction vessel fluid inlet of the reaction vessel to ensure a gas-tight/liquid-tight connection between the reaction vessel fluid inlet of the reaction vessel and the reaction mixture channel 3017 of the housing when the reaction vessel is coupled to the housing of the sample processing apparatus. In some instances, a groove can be provided near the reaction vessel fluid inlet of the reaction vessel and/or on the reaction vessel interface of the housing to receive therein the elastic sealing member. The elastic sealing member can have a sealing feature (e.g., protrusion) that seals in both radial and axial directions, thereby achieving a liquid sealing in both axial and radial directions.

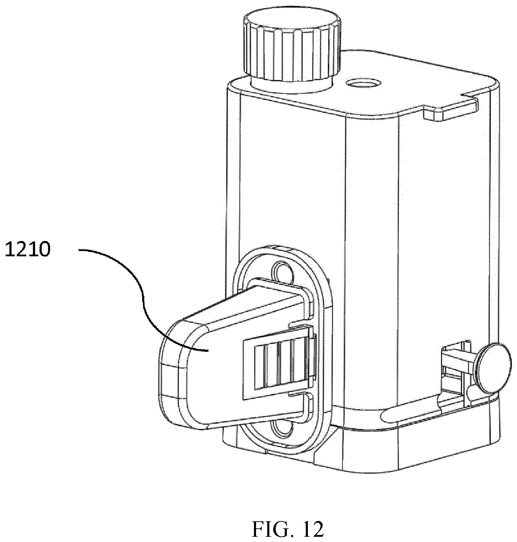

In some instances, a cap 1210 can be provided to cover the reaction vessel, as shown in FIG. 12. The cap can be removable. For example, prior to coupling the reaction vessel to a thermal cycling apparatus for PCR reaction, the cap can remain covering the reaction vessel to prevent operators or other objects from touching the reaction vessel. Once the reaction vessel is coupled to the thermal cycling apparatus to perform the PCR reaction, the cap can be removed.

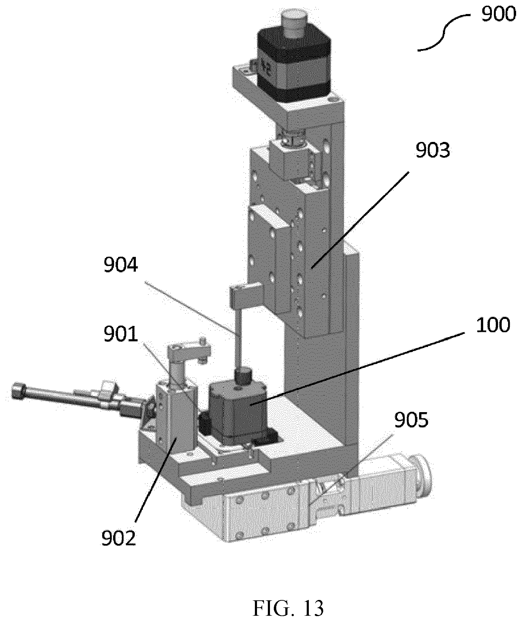

The sample processing apparatus of the present disclosure can be operated by an automated consumable operating mechanism to realize an automated processing of the sample. FIG. 13 depicts a consumable operating mechanism mating with an exemplary sample processing apparatus of the present disclosure. As shown in FIG. 13, the consumable operating mechanism 900 can comprise a positioning assembly 901 and a pressing assembly 902. The positioning assembly 901 can be configured to accurately position the sample processing apparatus 100 of the present disclosure at a preset position of an operating platform of the consumable operating mechanism and prevent the sample processing apparatus from moving in a horizontal direction. The pressing assembly 902 can be configured to press the sample processing apparatus 100 of the present disclosure at a preset position on the operating platform of the consumable operating mechanism to prevent the sample processing apparatus from moving in a vertical direction. The consumable operating mechanism can further comprise a plunger rod push-pull mechanism 903 and a plunger rod 904 coupled to the plunger rod push-pull mechanism 903. The plunger rod push-pull mechanism can be driven by a motor to control a motion of the plunger rod in a vertical direction. An accuracy in a movement of the plunger rod in the vertical direction, driven by the plunger rod push-pull mechanism, can be at least 1 mm, 0.5 mm, 0.1 mm, 50 microns (µm), 40 µm, 30 µm, 20 µm, 10 µm, 5 µm, 4 µm, 3 µm, or 1 µm . The translation speed of the plunger rod in the vertical direction is at least 1 mm/s, 2 mm/s, 3 mm/s, 4 mm/s, 5 mm/s, 6 mm/s, 7 mm/s, 8 mm/ s, 9 mm/s, 10 mm/s, 11 mm/s, 12 mm/s, 13 mm/s, 14 mm/s, 15 mm/s, 16 mm/s, 17 mm/s, 18 mm/ s, 19 mm/s, 20 mm/s, 25 mm/s, 30 mm/s, 35 mm/s, 40 mm/s, 45 mm/s or 50 mm/s. A thrust force of the plunger rod in the vertical direction can be at least 50 Newton (N), 60 N, 70 N, 80 N, 90 N, 100 N, 110 N, 120 N, 130 N, 140 N, 150 N, 160 N, 170 N, 180 N, 190 N or 200 N.

Once the sample processing apparatus of the present disclosure is positioned at a preset position of the operating platform of the consumable operating mechanism, the plunger rod can be accurately inserted into the fluid displacement chamber of the valve body of the sample processing apparatus and coupled with the pressure adjustment member (e.g., a plunger) in the fluid displacement chamber of the valve body. The consumable operating mechanism can further comprise a valve rotating mechanism 905 configured to be driven by a motor. The valve rotating mechanism can be coupled with the base portion of the valve body of the sample processing apparatus, such that the base phase of the valve body can be rotated. In some instances, the valve rotating mechanism can comprise a coupling feature (e.g., a protrusion or groove) that mates with the valve mating portion on the base portion of the sample processing apparatus, thereby rotating the base portion of the valve body of the sample processing apparatus with respect to the housing of the sample processing apparatus. An accuracy in rotation of the base portion of the valve body of the sample processing apparatus, driven by the valve rotating mechanism, can be at least 5 degrees, 4 degrees, 3 degrees, 2 degrees, 1 degree, 0.5 degree, 0.1 degree, 0.05 degree or 0.01 degree of central angle, enabling a precise alignment of the fluid port on the base portion of the valve body of the sample processing apparatus with the bottom opening of each chamber of the sample processing apparatus. A rotational speed of the base portion of the valve body of the sample processing apparatus, driven by the valve rotating mechanism, can be at least 10 degrees/second, 20 degrees/second, 30 degrees/second, 40 degrees/second, 50 degrees/second, 60 degrees/second, 70 degrees/second, 80 degrees/second, 90 degrees/second, 100s degree/second, 110 degrees/second, 120 degrees/second, 130 degrees/second, 140 degrees/second, 150 degrees/second, 160 degrees/second, 170 degrees/second or 180 degrees/second. A torque on the base portion of the valve body of the sample processing apparatus, driven by the valve rotating mechanism, can be at least 1 Newton meter (Nm), 2 Nm, 3 Nm, 4 Nm, 5 Nm, 6 Nm, 7 Nm, 8 Nm, 9 Nm, 10 Nm, 11 Nm, 12 Nm, 13 Nm, 14 Nm, 15 Nm, 16 Nm, 17 Nm, 18 Nm, 19 Nm or 20 Nm.

The consumable operating mechanism can be configured to operate the sample processing apparatus based on a preset workflow. A workflow of the consumable operating mechanism can vary based on a type of biological sample to be assayed, parameters to be assayed and a model of the sample processing apparatus. An operator can manually input parameters for the workflow. Alternatively, the operator can select an appropriate parameters for the workflow from a series of preset parameters. Alternatively, the operator can scan an information code (for example, barcode, QR code) attached to the sample processing apparatus and/or an lab request form to receive requirements on sample processing and/or reaction, and a control system can automatically select an appropriate parameters for the workflow from a series of preset parameters. Alternatively, the system can automatically scan an information code (for example, barcode, QR code) attached to the sample processing apparatus and/or the lab request form to receive requirements on sample processing and/or reaction and automatically select the appropriate parameters for the workflow from a series of preset parameters.

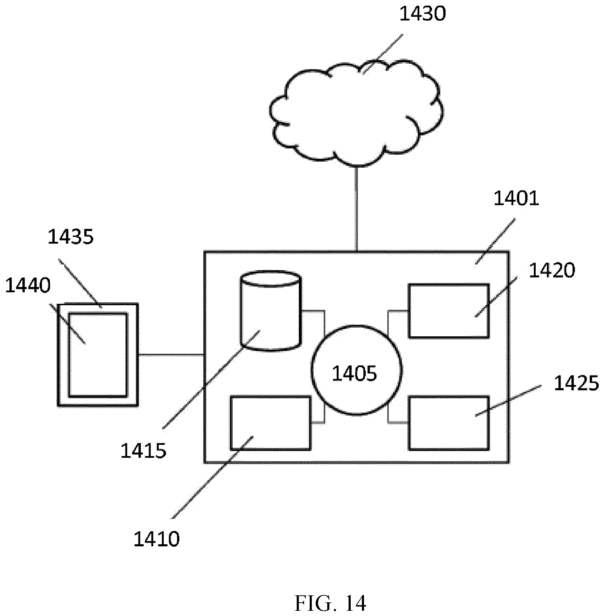

The present disclosure provides computer systems that are programmed to implement methods described herein. FIG. 14 shows a computer system 1401 programmed or otherwise configured to implement a sample processing method/detection method provided by the present disclosure. The computer system 1401 can process various aspects of the method of the present disclosure. The computer system 1401 can be an electronic device of a user or a computer system that is remotely located with respect to the electronic device. The electronic device can be a mobile electronic device.

The computer system 1401 comprises a central processing unit (CPU, also "processor" and "computer processor" herein) 1405, which can be a single core or multi core processor, or a plurality of processors for parallel processing. The computer system 1401 also comprises memory or memory location 1410 (e.g., random-access memory, read-only memory, flash memory), electronic storage unit 1415 (e.g., hard disk), communication interface 1420 (e.g., network adapter) for communicating with one or more other systems, and peripheral devices 1425, such as cache, other memory, data storage and/or electronic display adapters. The memory 1410, storage unit 1415, interface 1420 and peripheral devices 1425 are in communication with the CPU 1405 through a communication bus (solid lines), such as a motherboard. The storage unit 1415 can be a data storage unit (or data repository) for storing data. The computer system 1401 can be operatively coupled to a computer network ("network") 1430 with the aid of the communication interface 1420. The network 1430 can be the Internet, an internet and/or extranet, or an intranet and/or extranet that is in communication with the Internet. The network 1430 In some instances is a telecommunication and/or data network. The network 1430 can include one or more computer servers, which can enable distributed computing, such as cloud computing. The network 1430, In some instances with the aid of the computer system 1401, can implement a peer-to-peer network, which can enable devices coupled to the computer system 1401 to behave as a client or a server.

The CPU 1405 can execute a sequence of machine-readable instructions, which can be embodied in a program or software. The instructions can be stored in a memory location, such as the memory 1410. The instructions can be directed to the CPU 1405, which can subsequently program or otherwise configure the CPU 1405 to implement methods of the present disclosure. Examples of operations performed by the CPU 1405 can include fetch, decode, execute, and writeback.

The CPU 1405 can be part of a circuit, such as an integrated circuit. One or more other components of the system 1401 can be included in the circuit. In some instances, the circuit is an application specific integrated circuit (ASIC).

The storage unit 1415 can store files, such as drivers, libraries and saved programs. The storage unit 1415 can store user data, e.g., user preferences and user programs. The computer system 1401 In some instances can include one or more additional data storage units that are external to the computer system 1401, such as located on a remote server that is in communication with the computer system 1401 through an intranet or the Internet.

The computer system 1401 can communicate with one or more remote computer systems through the network 1430. For instance, the computer system 1401 can communicate with a remote computer system of a user. Examples of remote computer systems include personal computers (e.g., portable PC), slate or tablet PC's (e.g., Apple^{®} iPad, Samsung^{®} Galaxy Tab), telephones, Smart phones (e.g., Apple^{®} iPhone, Android-enabled device, Blackberry^{®}), or personal digital assistants. The user can access the computer system 1401 via the network 1430.

Methods as described herein can be implemented by way of machine (e.g., computer processor) executable code stored on an electronic storage location of the computer system 1401, such as, for example, on the memory 1410 or electronic storage unit 1415. The machine-executable or machine-readable code can be provided in the form of software. During use, the code can be executed by the processor 1405. In some instances, the code can be retrieved from the storage unit 1415 and stored on the memory 1410 for ready access by the processor 1405. In some instances, the electronic storage unit 1415 can be precluded, and machine-executable instructions are stored on memory 1410.

The code can be pre-compiled and configured for use with a machine having a processer adapted to execute the code or can be interpreted or compiled during runtime. The code can be supplied in a programming language that can be selected to enable the code to execute in a pre-compiled, interpreted, or as-compiled fashion.

Aspects of the systems and methods provided herein, such as the computer system 1401, can be embodied in programming. Various aspects of the technology can be thought of as "products" or "articles of manufacture" typically in the form of machine (or processor) executable code and/or associated data that is carried on or embodied in a type of machine-readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which can provide non- transitory storage at any time for the software programming. All or portions of the software can at times be communicated through the Internet or various other telecommunication networks. Such communications, for example, can enable loading of the software from one computer or processor into another, for example, from a management server or host computer into the computer platform of an application server. Thus, another type of media that can bear the software elements comprises optical, electrical and electromagnetic waves, such as used across physical interfaces between local devices, through wired and optical landline networks and over various air-links. The physical elements that carry such waves, such as wired or wireless links, optical links or the like, also can be considered as media bearing the software. As used herein, unless restricted to non-transitory, tangible "storage" media, terms such as computer or machine "readable medium" refer to any medium that participates in providing instructions to a processor for execution.

Hence, a machine-readable medium, such as computer-executable code, can take many forms, including but not limited to, a tangible storage medium, a carrier wave medium or physical transmission medium. Non-volatile storage media include, for example, optical or magnetic disks, such as any of the storage devices in any computer(s) or the like, such as can be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media can take the form of electric or electromagnetic signals, or acoustic or light waves such as those generated during radio frequency (RF) and infrared (IR) data communications. Common forms of computer- readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer can read programming code and/or data. Many of these forms of computer readable media can be involved in carrying one or more sequences of one or more instructions to a processor for execution.

The computer system 1401 can include or be in communication with an electronic display 1435 that comprises a user interface (UI) 1440 for providing, for example, a nucleic acid sequence, an enriched nucleic acid sample, a methylation profile, an expression profile, and an analysis of a methylation or expression profile. Examples of UI's include, without limitation, a graphical user interface (GUI) and web-based user interface.

### EXAMPLES

### Example 1: Sample processing apparatus having one sample chamber and three reagent chambers

In an exemplary embodiment, a sample chamber and three reagent chambers can be provided within the housing of the sample processing apparatus. A sample preservation solution can be prefilled in the sample chamber. After biological sampling, the sampling swab can be directly disposed into the sample chamber to contact the sampling swab with the sample preservation solution. A first reagent chamber can be a processing solution chamber in which a sample processing solution is prefilled. The sample processing solution and a biological sample can be proportionally mixed to produce a processed sample. A second reagent chamber can be a reconstitution buffer chamber in which a reconstitution buffer is prefilled. A third reagent chamber can be a solid reagent chamber in which a solid reagent such as PCR reaction solution lyophilized powder or reagent pellet is preloaded. The PCR reaction solution lyophilized powder can comprises, for example, RT enzyme, taq enzyme and primer probe to prepare a PCR reaction solution. The PCR reaction solution lyophilized powder can be dissolved with the reconstitution buffer and then mixed with the processed sample which is prepared with the a sample processing solution in the first reagent chamber. In this way, a reaction mixture for performing a PCR amplification reaction can be prepared.

In an exemplary embodiment, an exemplary workflow for processing a sample using the sample processing apparatus of the present disclosure can comprise the following steps.

Step 1. The first step can comprise removing the removable lid of the sample chamber of the sample processing apparatus, disposing a swab containing a biological sample (for example, a throat swab or a nasal swab) into the sample chamber, and tightening the lid. The filter provided in the sample chamber can filter out large particulate matters such as impurities in the biological sample. Optionally, the sample processing apparatus can be placed in a centrifuge for a centrifugation. With the centrifugation, the liquid in each chamber of the sample processing apparatus can be displaced to the bottom of each chamber. For example, the liquid can be displaced immediately adjacent to the through hole of the bottom seal shown in FIG. 3, such that the liquid in each chamber can be driven into the fluid channel in the base portion of the valve body via the through hole of the bottom seal.

Step 2: The second step can comprise positioning and securing the sample processing apparatus on the consumable operating mechanism and operating the consumable operating mechanism based on a preset workflow, such that a preset sample processing is performed in the sample processing apparatus.

In an exemplary embodiment, operations on the sample processing apparatus by the consumable operating mechanism can comprise the following processes.
(a) With the aid of the positioning assembly and the pressing assembly of the consumable operating mechanism, the sample processing apparatus can be fixed at a preset position on the operating platform of the consumable operating mechanism, such that movements of the sample processing apparatus in both horizontal and vertical directions can be avoid.
(b) The plunger rod of the plunger rod push-pull mechanism of the consumable operating mechanism can be inserted into the fluid displacement chamber of the valve body of the sample processing apparatus and coupled with the pressure adjustment member (e.g., a plunger) in the fluid displacement chamber of the valve body. In an initial status of the sample processing apparatus , the fluid displacement chamber can be fluidically isolated from the sample chamber or any reagent chamber of the valve body. In some instances, the fluid port on the base portion of the valve body of the sample processing apparatus is not aligned with the bottom opening of any chamber in the sample processing apparatus. In some instances, the fluid port on the base portion of the valve body of the sample processing apparatus can be aligned with the reaction mixture channel 3017 in the housing of the sample processing apparatus.
(c) The valve rotating mechanism of the consumable operating mechanism can be coupled to the base portion of the valve body of the sample processing apparatus (for example, to the valve mating portion provided on the base portion of the valve body). The base portion of the valve body of the sample processing apparatus can be rotated (e.g., clockwise) with an angle to align the fluid port on the base portion of the valve body with the bottom opening of the sample chamber.
(d) The plunger rod of the plunger rod push-pull mechanism of the consumable operating mechanism can move the plunger in the fluid displacement chamber of the valve body of the sample processing apparatus upward. The upward movement of the plunger in the fluid displacement chamber of the valve body can decrease a pressure in the fluid displacement chamber of the valve body, thereby transferring (e.g., drawing) an amount of biological sample from the sample chamber of the sample processing apparatus into the fluid displacement chamber of the valve body through the bottom opening of the sample chamber, the fluid port on the base portion of the valve body and the fluid channel in the base portion of the valve body .
(e) The valve rotating mechanism of the consumable operating mechanism can rotate the base portion of the valve body of the sample processing apparatus clockwise with an angle to align the fluid port on the base portion of the valve body with the bottom opening of the processing solution chamber.
(f) The plunger rod of the plunger rod push-pull mechanism of the consumable operating mechanism can move the plunger in the fluid displacement chamber of the valve body of the sample processing apparatus downward. The downward movement of the plunger in the fluid displacement chamber of the valve body can increase the pressure in the fluid displacement chamber of the valve body, thereby transferring (e.g., pressing) an amount of the biological sample from the fluid displacement chamber of the valve body into the processing solution chamber through the fluid channel in the base portion of the valve body, the fluid port on the base portion of the valve body and the bottom opening of the processing solution chamber. In the processing solution chamber, the biological sample can be mixed with the prefilled sample processing solution.
(g) The plunger rod of the plunger rod push-pull mechanism of the consumable operating mechanism can move the plunger in the fluid displacement chamber of the valve body of the sample processing apparatus upward, thereby drawing an amount of liquid, which is a mixture of the biological sample and the sample processing solution, from the processing solution chamber of the sample processing apparatus into the fluid displacement chamber of the valve body of the sample processing apparatus.
(h) The valve rotating mechanism of the consumable operating mechanism can rotate the base portion of the valve body of the sample processing apparatus clockwise with an angle to align the fluid port on the base portion of the valve body with the bottom opening of the reconstitution buffer chamber.
(i) The plunger rod of the plunger rod push-pull mechanism of the consumable operating mechanism can move the plunger in the fluid displacement chamber of the valve body of the sample processing apparatus downward, thereby driving an amount of liquid, which is a mixture of the biological sample and the sample processing solution, from the fluid displacement chamber of the valve body, into the reconstitution buffer chamber. In the reconstitution buffer chamber, the mixture of the biological sample and the sample processing solution can be mixed with the prefilled reconstitution buffer.
(j) The plunger rod of the plunger rod push-pull mechanism of the consumable operating mechanism can move the plunger in the fluid displacement chamber of the valve body of the sample processing apparatus upward, thereby drawing an amount of liquid, which is a mixture of the biological sample, the sample processing solution and the reconstitution buffer, from the reconstitution buffer chamber into the fluid displacement chamber.
(k) The valve rotating mechanism of the consumable operating mechanism can rotate the base portion of the valve body of the sample processing apparatus clockwise at an angle to align the fluid port on the base portion of the valve body with the bottom opening of the solid reagent chamber.
(l) The plunger rod of the plunger rod push-pull mechanism of the consumable operating mechanism can move the plunger in the fluid displacement chamber of the valve body of the sample processing apparatus downward, thereby forcing an amount of liquid, which is a mixture of the biological sample, the sample processing solution and the reconstitution buffer, from the fluid displacement chamber of the valve body into the solid reagent chamber. In the solid reagent chamber, the liquid mixture dissolves the prefilled lyophilized powder.
(m) The plunger rod of the plunger rod push-pull mechanism of the consumable operating mechanism can move the plunger in the fluid displacement chamber of the valve body of the sample processing apparatus upward, thereby drawing a certain amount of liquid, which is a mixture of the biological sample, the sample processing solution, the reconstitution buffer and the dissolved lyophilized powder, from the solid reagent chamber into the fluid displacement chamber.
(n) The valve rotating mechanism of the consumable operating mechanism can rotate the base portion of the valve body of the sample processing apparatus clockwise at an angle to align the fluid port on the base portion of the valve body with a reaction mixture channel in the sample processing apparatus. As described hereinabove with reference to FIG. 8, the reaction mixture channel 3017 in the sample processing apparatus can be in fluidic communication with the reaction vessel fluid inlet of the reaction vessel 201.
(o) The plunger rod of the plunger rod push-pull mechanism of the consumable operating mechanism can move the plunger in the fluid displacement chamber of the valve body of the sample processing apparatus downward, thereby driving an amount of liquid, which is a mixture of the biological sample, the sample processing solution, the reconstitution buffer and the dissolved lyophilized powder, from the fluid displacement chamber into the reaction region in the reaction vessel 201. Once (o) is completed, the biological sample can be processed and the fluid can be transferred.
(p) Once the predetermined reaction (for example, an amplification reaction) and detection are completed, the valve rotating mechanism of the consumable operating mechanism can rotate the base portion of the valve body of the sample processing apparatus clockwise at an angle to disengage the fluid port in the base portion of the valve body from the reaction mixture channel in the housing of the sample processing apparatus.
(q) The plunger rod of the plunger rod push-pull mechanism of the consumable operating mechanism can move the plunger in the fluid displacement chamber of the valve body of the sample processing apparatus to upward, such that the plunger rod of the consumable operating mechanism is decoupled and disengaged with the pressure adjustment member (e.g., a plunger) in the fluid displacement chamber of the valve body. Therefore, the sample processing apparatus can be removed from the consumable operating mechanism.

In another exemplary embodiment, operations on the sample processing apparatus by the consumable operating mechanism can comprise the following processes.
(a1) With the aid of the positioning assembly and the pressing assembly of the consumable operating mechanism, the sample processing apparatus can be fixed at a preset position of the operating platform of the consumable operating mechanism, such that movements of the sample processing apparatus in both horizontal and vertical directions can be avoid.
(b 1) The plunger rod of the plunger rod push-pull mechanism of the consumable operating mechanism can be inserted into the fluid displacement chamber of the valve body of the sample processing apparatus and coupled with the pressure adjustment member (e.g., a plunger) in the fluid displacement chamber of the valve body.
(c1) The valve rotating mechanism of the consumable operating mechanism can be coupled to the base portion of the valve body of the sample processing apparatus (for example, to the valve mating portion provided on the base portion of the valve body). The base portion of the valve body of the sample processing apparatus can be rotated (e.g., clockwise) with an angle to align the fluid port on the base portion of the valve body with the bottom opening of the sample chamber.
(d1) The plunger rod of the plunger rod push-pull mechanism of the consumable operating mechanism can move the plunger in the fluid displacement chamber of the valve body of the sample processing apparatus upward, thereby drawing an amount of biological sample from the sample chamber of the sample processing apparatus into the fluid displacement chamber of the valve body.
(e1) The valve rotating mechanism of the consumable operating mechanism can rotate the base portion of the valve body of the sample processing apparatus clockwise with an angle to align the fluid port on the base portion of the valve body with the bottom opening of the processing solution chamber.
(f1) The plunger rod of the plunger rod push-pull mechanism of the consumable operating mechanism can move the plunger in the fluid displacement chamber of the valve body of the sample processing apparatus upward further, thereby drawing an amount of the sample processing solution from the processing solution chamber into the fluid displacement chamber of the valve body of the sample processing apparatus. Once (f1) is completed, the liquid in the fluid displacement chamber of the valve body can be a mixture of the biological sample and the sample processing solution.
(g1) The valve rotating mechanism of the consumable operating mechanism can rotate the base portion of the valve body of the sample processing apparatus clockwise with an angle to align the fluid port on the base portion of the valve body with the bottom opening of the reconstitution buffer chamber.
(h1) The plunger rod of the plunger rod push-pull mechanism of the consumable operating mechanism can move the plunger in the fluid displacement chamber of the valve body of the sample processing apparatus upward further, thereby drawing an amount of the reconstitution buffer from the reconstitution buffer chamber into the fluid displacement chamber of the valve body of the sample processing apparatus. Once (h1) is completed, the liquid in the fluid displacement chamber of the valve body can be a mixture of the biological sample, the sample processing solution and the reconstitution buffer.
(i1) The valve rotating mechanism of the consumable operating mechanism can rotate the base portion of the valve body of the sample processing apparatus clockwise at an angle to align the fluid port on the base portion of the valve body with the bottom opening of the solid reagent chamber.
(j 1) The plunger rod of the plunger rod push-pull mechanism of the consumable operating mechanism can move the plunger in the fluid displacement chamber of the valve body of the sample processing apparatus downward, thereby transferring at least portion of the liquid in the fluid displacement chamber into the solid reagent chamber and dissolve the solid reagent (such as lyophilized powder or reagent pellets) in the solid reagent chamber. Subsequently, the plunger rod of the plunger rod push-pull mechanism of the consumable operating mechanism can move the plunger in the fluid displacement chamber of the valve body of the sample processing apparatus upward, thereby drawing at least portion of the liquid in which the solid reagent is dissolved from the solid reagent chamber into the fluid displacement chamber of the valve body of the sample processing apparatus. A liquid mixture in the fluid displacement chamber can dissolve the lyophilized powder. Once (j 1) is completed, the liquid in the fluid displacement chamber of the valve body can be a mixture of the biological sample, the sample processing solution, the reconstitution buffer and the dissolved lyophilized powder.
(k1) The valve rotating mechanism of the consumable operating mechanism can rotate the base portion of the valve body of the sample processing apparatus clockwise at an angle to align the fluid port on the base portion of the valve body with a reaction mixture channel in the sample processing apparatus.
(11) The plunger rod of the plunger rod push-pull mechanism of the consumable operating mechanism can move the plunger in the fluid displacement chamber of the valve body of the sample processing apparatus downward, thereby driving an amount of liquid from the fluid displacement chamber into the reaction region in the reaction vessel 201. Once (11) is completed, the biological sample can be processed and the fluid can be transferred.

### Example 2: Sample processing apparatus having one sample chamber and two reagent chambers

In another exemplary embodiment, a sample chamber and two reagent chambers can be provided within the housing of the sample processing apparatus. At least one or both of a sample preservation solution and a sample processing solution can be prefilled in the sample chamber. After biological sampling, the sampling swab can be directly disposed into the sample chamber to contact with at least one or both of the sample preservation solution and the sample processing solution. A first reagent chamber can be a reconstitution buffer chamber in which a reconstitution buffer is prefilled. A second reagent chamber can be a solid reagent chamber in which a solid reagent such as PCR reaction solution lyophilized powder is preloaded. The PCR reaction solution lyophilized powder can be dissolved with the reconstitution buffer and then mixed with the processed sample which is prepared with at least one of the sample preservation solution and the sample processing solution in the sample chamber. In this way, a reaction mixture for performing a PCR amplification reaction can be prepared.

The operations on the sample processing apparatus using the consumable operating mechanism in Example 2 can be similar to those operations described in Example 1. Since the sample processing apparatus has less reagent chamber, the corresponding rotation operation of the base portion of the valve body of the sample processing apparatus by the valve rotating mechanism of the consumable operating mechanism and the fluid transfer operation can be omitted accordingly.

### Example 3: Sample processing apparatus having one sample chamber and no reagent chamber

In yet another exemplary embodiment, the housing of the sample processing apparatus comprises one sample chamber without any reagent chamber. The sample chamber can be prefilled with all reagents required for processing a biological sample. After biological sampling, the sampling swab can be directly disposed into the sample chamber to contact the sampling swab with the liquid reagent. In this way, a reaction mixture for performing a PCR amplification reaction can be prepared.

Operations on the sample processing apparatus by the consumable operating mechanism can comprise the following processes.
(1) With the aid of the positioning assembly and the pressing assembly of the consumable operating mechanism, the sample processing apparatus can be fixed at a preset position on the operating platform of the consumable operating mechanism, such that movements of the sample processing apparatus in both horizontal and vertical directions can be avoid.
(2) The plunger rod of the plunger rod push-pull mechanism of the consumable operating mechanism can be inserted into the fluid displacement chamber of the valve body of the sample processing apparatus and coupled with the pressure adjustment member (e.g., a plunger) in the fluid displacement chamber of the valve body.
(3) The valve rotating mechanism of the consumable operating mechanism can rotate the base portion of the valve body of the sample processing apparatus at an angle to align the fluid port on the base portion of the valve body with a reaction mixture channel in the sample processing apparatus.
(4) The plunger rod of the plunger rod push-pull mechanism of the consumable operating mechanism can move the plunger in the fluid displacement chamber of the valve body of the sample processing apparatus downward, thereby driving an amount of liquid from the fluid displacement chamber into the reaction region in the reaction vessel 201. Once (4) is completed, the biological sample can be processed and the fluid can be transferred.

### Example 4: Sample processing apparatus having one sample chamber and five reagent chambers

In an exemplary embodiment, a sample chamber and six reagent chambers can be provided within the housing of the sample processing apparatus. The six reagent chambers can comprise a nucleic acid extraction chamber, a rinsing solution chamber, a reconstitution buffer chamber, an anti-adherence rinsing solution chamber and a lyophilized pellet chamber. A lysis solution can be prefilled in the nucleic acid extraction chamber. A rinsing solution can be prefilled in the rinsing solution chamber. A reconstitution buffer can be prefilled in the reconstitution buffer chamber. A anti-adherence rinsing solution can be prefilled in the anti-adherence rinsing solution chamber. Lyophilized pellets can be preloaded in the lyophilized pellet chamber. In some instances, magnetic beads can be additionally preloaded in the nucleic acid extraction chamber for nucleic acid extraction using magnetic beads.

Step 1: At least a portion of the biological sample can be drawn from the sample chamber and transferred to the nucleic acid extraction chamber where the portion of the biological sample can be fully mixed with the lysis solution in the nucleic acid extraction chamber to lyse the pathogens in the biological sample. In the meantime, magnetic beads in the nucleic acid extraction chamber can simultaneously adsorb the nucleic acid substance released by the lysis. Then, an external magnetic field can be applied to the magnetic beads in the nucleic acid extraction chamber through a magnetic permeable component, such that the magnetic beads are adsorbed to an inner wall of the nucleic acid extraction chamber. Subsequently, liquid in the nucleic acid extraction chamber can be transferred to a waste liquid chamber.

Step 2: The rinsing solution can be drawn from the rinsing solution chamber to the nucleic acid extraction chamber, to rinse and remove impurities adsorbed by the magnetic beads. A waste liquid left by rinsing can be transferred to the waste liquid chamber.

Step 3: The reconstitution buffer can be drawn and transferred to the nucleic acid extraction chamber, thereby eluting the nucleic acid substance from the magnetic beads.

Step 4: An external magnetic field can be applied to the magnetic beads in the nucleic acid extraction chamber through the magnetic permeable component, such that the magnetic beads are immobilized at the inner wall of the nucleic acid extraction chamber. Subsequently, the reconstitution buffer in which the nucleic acid substance is eluted can be transferred to the lyophilized pellet chamber where the lyophilized pellets can be completely dissolved.

Step 5: The reaction mixture in which the lyophilized pellets are dissolved can be transferred to the reaction chamber to perform the PCR reaction.

From the principles taught in the above embodiments, those skilled in the art can understand that a sample chamber and any number of reagent chambers can be provided in the housing of the sample processing apparatus. The number of reagent chambers can be determined based on a type and/or quantity of biological samples to be processed and/or a type and/or number of reactions to be performed. For example, the number of reagent chambers in the housing of the sample processing apparatus can be 0, 1, 2, 3, 4, 5, 6, 7, 8, 9, 10 or more.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein can be employed in practicing the invention. It is intended that the following claims define the scope of the invention and that methods and structures within the scope of these claims and their equivalents be covered thereby.

## Claims

1. A sample processing apparatus, comprising:
a housing having at least one chamber; and
a valve body coupled to the housing, the valve having a fluid displacement chamber, no more than one fluid port and no more than one fluid channel, the no more than one fluid channel fluidically connecting the fluid displacement chamber and the fluid port, the fluid displacement chamber being coupled to a pressure adjustment member which is configured to change a pressure within the fluid displacement chamber,
wherein the valve body is adjustable with respect to the housing such that the fluid port of the valve body is selectively in fluidic communication with the at least one chamber of the housing.

2. The sample processing apparatus of claim 1, wherein the at least one chamber comprises a sample chamber configured to receive therein a biological sample.

3. The sample processing apparatus of claim 2, wherein the sample chamber is configured to contain therein at least one reagent for processing the biological sample.

4. The sample processing apparatus of claim 2, wherein the sample chamber is provided with a removable seal which seals an opening end of the sample chamber.

5. The sample processing apparatus of claim 2, wherein the sample chamber comprises a filter.

6. The sample processing apparatus of claim 2, wherein the at least one chamber further comprises at least one reagent chamber configured to contain therein at least one reagent for processing the biological sample.

7. The sample processing apparatus of claim 6, wherein the at least one reagent comprises a solid reagent or a liquid reagent.

8. The sample processing apparatus of claim 6, wherein the at least one reagent chamber is configured to contain therein magnetic beads, the magnetic beads being configured to facilitate an nucleic acid extraction of the biological sample.

9. The sample processing apparatus of claim 8, wherein the at least one reagent chamber containing the magnetic beads is magnetically coupled to an external magnetic field.

10. The sample processing apparatus of claim 9, wherein the at least one reagent chamber containing the magnetic beads is magnetically coupled to an external magnetic field through a component having a high magnetic permeability.

11. The sample processing apparatus of claim 1, wherein the at least one chamber comprises a reserved chamber in which no reagent is contained.

12. The sample processing apparatus of claim 1, wherein the at least one chamber is sealed.

13. The sample processing apparatus of claim 1, wherein the valve body is rotatable relative to the housing.

14. The sample processing apparatus of claim 1, wherein the pressure adjustment member comprises a plunger or a piston.

15. The sample processing apparatus of claim 1, wherein a decrease in the pressure within the fluid displacement chamber draws fluid into the fluid displacement chamber, and wherein an increase in the pressure within the fluid displacement chamber expels fluid from the fluid displacement chamber.

16. The sample processing apparatus of claim 1, further comprising a reaction vessel configured to removably couple to the housing.

17. The sample processing apparatus of claim 16, wherein the fluid port of the valve body is selectively in fluidic communication with the reaction vessel.

18. The sample processing apparatus of claim 16, wherein the reaction vessel comprises a first port, a second port and a reaction region in fluidic communication with the first port and the second port.

19. The sample processing apparatus of claim 18, wherein the reaction region comprises a first side and a second side, the first side and the second side comprise a membrane.

20. The sample processing apparatus of claim 18, wherein the second port is sealed with an inflatable membrane.

21. The sample processing apparatus of claim 16, wherein the reaction vessel is dimensioned and shaped to be operated with an apparatus configured to perform an nucleic acid amplification or an nucleic acid detection.

22. The sample processing apparatus of claim 1, further comprising a cap configured to cover the reaction vessel.

23. A method for processing a sample, comprising:
providing the sample processing apparatus of claim 1;
coupling the pressure adjustment member to the fluid displacement chamber, the pressure adjustment member being operated to change a pressure within the fluid displacement chamber; and
adjusting a position of the valve body with respect to the housing, such that the fluid port of the valve body is selectively in fluidic communication with the at least one chamber in the housing.

24. The method of claim 23, further comprising placing a biological sample into a sample chamber.

25. The method of claim 24, further comprising:
adjusting a position of the valve body with respect to the housing, such that the fluid port of the valve body is in fluidic communication with the sample chamber in the housing; and
operating the pressure adjustment member to transfer a solution comprising at least a portion of the biological sample into the fluid displacement chamber.

26. The method of claim 24, further comprising:
adjusting a position of the valve body with respect to the housing, such that the fluid port of the valve body is in fluidic communication with the at least one chamber in the housing; and
operating the pressure adjustment member to transfer a solution comprising at least a portion of the biological sample into the at least one chamber.

27. The method of claim 26, further comprising:
adjusting a position of the valve body with respect to the housing, such that the fluid port of the valve body is in fluidic communication with a reaction vessel; and
operating the pressure adjustment member to transfer a solution comprising at least a portion of the biological sample into the reaction vessel.
